# EUROPEAN PATENT SPECIFICATION

(11) **EP 3 880 834 B1**
(45) Date of publication and mention of the grant of the patent: **12.10.2022**
(21) Application number: 19802186.7
(22) Date of filing: 15.11.2019
(51) Int. Cl.: C12P 5/02, C02F 11/04

(54) **A METHOD FOR PROCESSING BIOMASS DIGESTATE**
VERFAHREN ZUR VERARBEITUNG VON BIOMASSENGÄRRESTEN
PROCÉDÉ DE TRAITEMENT DE PRODUITS DE DIGESTION DE BIOMASSE

(30) Priority: 16.11.2018 EP 18206737
(43) Date of publication of application: 22.09.2021
(73) Proprietor: NGF Nature Energy Biogas A/S, 5220 Odense SØ (DK)
(72) Inventor: JEPPESEN, Martin Dan, 5200 Odense V (DK); GYLLENBORG, Morten Enzo, 5700 Svendborg (DK); NIELSEN, Sine Stylsvig, 5000 Odense C (DK)
(74) Representative: Patentgruppen A/S
(86) International application number: PCT/EP2019/081529
(87) International publication number: WO 2020/099657

(56) References cited:
- EP-A1- 3 162 898
- LUCIANO DIAS XAVIER ET AL: "Study of the recovery of phosphorus from struvite precipitation in supernatant line from anaerobic digesters of sludge", WATER SCIENCE AND TECHNOLOGY, vol. 69, no. 7, 4 February 2014 (2014-02-04), pages 1546-1551, XP055587570, Bristol (UK) ISSN: 0273-1223, DOI: 10.2166/wst.2014.033
- Hongjian Lin ET AL: "Phosphorus Removal and Recovery from Digestate after Biogas Production" In: "Biofuels - Status and Perspective", 30 September 2015 (2015-09-30), InTech, XP055587572, ISBN: 978-953-51-2177-0 pages 517-546, DOI: 10.5772/60474, the whole document
- KUDANGA T ET AL: "Laccase applications in biofuels production: Current status and future prospects", APPLIED MICROBIOLOGY AND BIOTECHNOLOGY AUGUST 2014,, vol. 98, no. 15, August 2014 (2014-08), pages 6525-6542, XP002782948, DOI: 10.1007/S00253-014-5810-8
- YI ZHENG ET AL: "Pretreatment of lignocellulosic biomass for enhanced biogas production", PROGRESS IN ENERGY AND COMBUSTION SCIENCE., vol. 42, June 2014 (2014-06), pages 35-53, XP055587938, NL ISSN: 0360-1285, DOI: 10.1016/j.pecs.2014.01.001

## Description

### FIELD OF INVENTION

The invention relates to a process for processing biomass digestate for obtaining e.g. biogas, lignin and nutrients like phosphorus and nitrogen.

### BACKGROUND

Biomass digestate is a product of digestion of biomass and represents the digested material, which is removed from the digestor after recovery of biogas.

Digestate has most often a rich content of nutrients including nitrogen (N), phosphorus (P), potassium (K) and sulfur (S). Digestate may thus be applied as fertilizer to crops without further processing, however this has the disadvantage that the concentration of e.g. phosphorus and nitrogen and especially their relative amount i.e. ratio may not be controlled or adjusted according to e.g. regulations or crop requirements. Furthermore, deposition of biomass digestate is costly. In addition, many valuable products present in the biomass digestate, such as organic matter that may be converted into biogas or purified lignin, is not utilized.

It is an object of the present invention to solve the above problems.

EP 3162898 A1 relates to the use of selected bacteria species of *Clostridium thermocellum* in the process of biogas production from a mixture of ingestate and digestate.

The application describes an optimum mixture of bacteria species used in an anaerobe digestion step of digestate for obtaining free sugars and lysed cellulose and/or hemicellulose.

However, the above mentioned problems remains to be solved.

### SUMMARY

The invention relates to a method for processing biomass digestate, the method comprising the steps of
- providing a biomass digestate,
- subjecting the biomass digestate to a separation step into a liquid fraction and a solid biomass digestate fraction,
- subjecting the solid biomass digestate fraction to an acidic wash step at a pressure below 200 kPa (2 bar) to obtain a washed biomass digestate,
- separating the washed biomass digestate into a liquid wash fraction and a solid wash fraction,
- posttreating the solid wash fraction to obtain a posttreated solid wash fraction and
- anaerobic digesting the posttreated solid wash fraction to obtain a posttreated biomass digestate

wherein the amount of phosphorus in the solid wash fraction is reduced compared to amount of phosphorus in the solid biomass digestate fraction,
wherein the posttreatment is selected from the list consisting of steam explosion, laccase treatment, base treatment, dilute acid treatment, thermal treatment, dissolution in ionic liquids and subsequent precipitation, dissolution in molten salt hydrates, or an organosolv process, or combinations thereof.

The present invention advantageously utilizes biomass digestate to obtain valuable products such as nutrients for agricultural use and high value products such as biogas and lignin.

A significant advantage of the invention is that a biomass digestate can be utilized to provide valuable products at a relatively low cost. This may reduce cost in biogas production, since a lesser amount of digestate is produced and a lesser amount of waste thus has to be disposed of, which is costly.

Another advantage of the invention is that nutrients, such as phosphorus and nitrogen, can be recovered individually, and that the individual concentrations of nutrients and especially their relative amount i.e. ratio may thus be controlled and adjusted according to e.g. regulations or crop requirements, when used as fertilizer. The liquid wash fraction, for example, comprises a high amount of the nutrient phosphorus originally contained in the biomass digestate and may thus be utilized as e.g. a biofertilizer in desired amounts.

A further advantage is that the digestibility of the biomass digestate may be improved resulting in quicker digestion.

The present invention also provides for significantly increasing the total amount of biogas obtainable from a certain amount of biomass. The methane production may according to the method of the invention in this way be increased without adding more biomass, as the biomass is better utilized. It may be increased with more than 10% or more than 20% or even more than 30%.

A further advantage of the invention is that the posttreated biomass digestate may comprise lignin of a high purity and quality, since lignin may in this way be produced with a substantially reduced amount of nutrients, such as P and N, and carbohydrates or even substantially free from nutrients and carbohydrates. Thus, providing a cleaner and higher value lignin. The lignin may be used e.g. as bio-pellets or as replacement for bitumen and other high value green chemicals.

A further advantage of the invention is that the waste product, i.e. the posttreated biomass digestate or the solid posttreated biomass digestate fraction or the dried posttreated biomass digestate, contains nutrients, such as phosphorus and nitrogen, in controlled relative amounts i.e. ratios, making the waste product applicable as fertilizer.

A further advantage of the invention is that the waste product, i.e. the posttreated biomass digestate or the solid posttreated biomass digestate fraction or the dried posttreated biomass digestate, contains nutrients, such as phosphorus and nitrogen, in controlled relative amounts i.e. ratios, making the solid waste product less costly to dispose.

A further advantage of the invention is that the waste product, i.e. the posttreated biomass digestate or the solid posttreated biomass digestate fraction or the dried posttreated biomass digestate, contains low amounts of nutrients such as phosphorus and nitrogen making the solid waste product less costly to dispose.

It has thus surprisingly been discovered by the inventors that by employing first an acidic wash step for removing and recovering phosphorus, and then posttreating the biomass digestate, high amounts of both phosphorus, biogas, nitrogen and lignin may be obtained in high purity.

According to an embodiment of the invention, the acidic wash step is performed at a pressure below 200 kPa (2 bar), such as below 180 kPa (1.8 bar), such as below 150 kPa (1.5 bar), such as below 130 kPa (1.3 bar), such as below 110 kPa (1.1 bar).

The acidic wash step may be performed at a pressure between 90 kPa (0.9 bar) and 200 kPa (2 bar), such as between 90 kPa (0.9 bar) and 180 kPa (1.8 bar), such as between 90 kPa (0.9 bar) and 150 kPa (1.5 bar), such as between 90 kPa (0.9 bar) and 130 kPa (1.3 bar), such as between 90 kPa (0.9 bar) and 110 kPa (1.1 bar).

The acidic wash step may be performed at a pressure between 50 kPa (0.5 bar) and 200 kPa (2 bar), such as between 60 kPa (0.6 bar) and 180 kPa (1.8 bar), such as between 70 kPa (0.7 bar) and 150 kPa (1.5 bar), such as between 80 kPa (0.8 bar) and 130 kPa (1.3 bar), such as between 90 kPa (0.9 bar) and 110 kPa (1.1 bar).

According to an advantageous embodiment of the invention, the acidic wash step is non-pressurized.

The acidic wash step may be performed at ambient pressure.

All of the above advantages are also obtained when the acidic wash step is non-pressurized, and in addition the method and especially the acidic wash step may be performed easier (for example in a continuous way) and cheaper, when the acidic wash step is non-pressurized, since it may be performed in an open system or may be performed in reactors with over-/underpressure valves. When the acidic wash step is non-pressurized, the severity of the acidic wash is also lower than when a higher pressure is applied, and thus milder conditions are obtained.

According to an embodiment of the invention, the amount of phosphorus in the solid wash fraction is less than 10% by weight of the phosphorus in the biomass digestate. By subjecting the solid fraction of biomass digestate to an acidic wash step a large proportion of phosphorus can be removed, such as more than 90%, or even more than 95 or 98%, or even substantially all the phosphorus that was present in the biomass digestate may be removed.

According to an embodiment of the invention, the pH in the acidic wash step is lower than 6.5, such as lower than 6, such as lower than 4.

The pH in the acidic wash step may be between 0 and lower than 7, such as between 0 and 6.5, such as between 0 and 6, such as between 0 and 4.

It has been shown by the present inventors that more phosphorus can be removed by lowering pH.

According to an embodiment of the invention, the temperature in the acidic wash step is between 50 to 100 degrees Celsius.

The acidic wash may be more effective at removing (reducing) phosphorus at a temperature of around 90 to 100 degrees Celsius or close thereto, however from an economical view point a lower temperature such as around 50 to 70 degrees Celsius may be more favorable. When the acidic wash step is performed at temperatures not higher than 100 degrees Celsius, it has the advantage to be able to be performed in an open continuous system. Temperatures above 100 degrees Celsius have the potential draw-back than a closed container with pressure is required, which means that it is not possible to run it in a continuous way. At temperatures above 100 degrees Celsius, compounds that inhibit the subsequent anaerobic digestion may also be formed.

The acidic wash step may be performed for between 10 minutes to 24 hours, such as between 10 minutes to 10 hours, such as between 10 minutes and 5 hours.

The acidic wash step can be effective in removing phosphorus by being performed in only a short time, such as e.g. 10 minutes, however increasing the incubation time i.e. the time the acidic wash step is performed can increase the amount of phosphorus removed from the solid biomass digestate fraction. If the acidic wash step is performed for a short time, the amount of phosphorus removed from the solid biomass digestate fraction may also be increased by raising the concentration of the acid or increasing the temperature.

The concentration of the acid in the acidic wash step may be between 0.01 to 1 mol/liter, such as between 0.04 to 0.5 mol/liter, such as between 0.04 to 0.2 mol/liter.

By increasing the concentration of acid more phosphorus may be removed. According to an embodiment of the invention, the acid in the acidic wash step is a strong acid.

The strong acid may e.g. be sulphuric acid, hydrochloric acid, or oxalic acid or any combination thereof.

According to an embodiment of the invention, the acid in the acidic wash step is a weak acid.

The weak acid may be a weak organic acid like e.g. acetic acid, citric acid, formic acid or lactic acid.

According to an embodiment of the invention, the acid in the acidic wash step is an organic acid. An advantage of using organic acids is that an organic acid constitutes a carbon source in biogas production i.e. the organic acid is converted into gas.

The acid in the acidic wash may be a divalent acid.

According to an embodiment of the invention, the amount of phosphorus in the liquid wash fraction is reduced to obtain a low phosphorus liquid fraction.

The amount of phosphorus in the liquid wash fraction may be reduced i.e. removed by more than 80% by weight, such as more than 90% by weight, such as more than 95% by weight or even substantially all the phosphorus may be removed from the liquid wash fraction.

According to an embodiment of the invention, the amount of phosphorus in the liquid wash fraction is reduced by subjecting the liquid wash fraction to ion exchange (IEX). By using ion exchange a selective removal of phosphorus, by e.g. not removing nitrogen, as well as very high amount of phosphorus may be removed, such as more than 95% and even more than 98% or even almost all the phosphorus present in the liquid wash fraction may be removed by ion exchange. Thus, ion exchange represents a very efficient and selective way of removing phosphorus from the liquid wash fraction and thus a corresponding high amount of phosphorus may be precipitated as e.g. Na₃PO₄ by subjecting the ion exchange resin to NaOH. The phosphorus that is removed from the liquid wash fraction may then be used as e.g. a fertilizer and has the advantage that it may be substantially free from other nutrients such as nitrogen.

The ion exchange may be performed by passing the liquid wash fraction over a resin.

The ion exchangers may e.g. be a strongly basic anion exchanger, such as Amberjet 4200 from Merck or an ion exchanger comprising a polystyrene-based resin, such as Lewatit FO 36 from Lanxess or a mixed bed ion exchanger such as Amberlite MB-3 from Merck.

The amount of phosphorus in the liquid wash fraction may also be reduced by nutrient recovery processes such as precipitation with base, such as precipitation with Ca(OH)₂, such as precipitation with NaOH, as struvite or by reverse osmosis.

According to an embodiment of the invention, the amount of phosphorus in the liquid wash fraction is reduced by subjecting the liquid wash fraction to base precipitation. According to an embodiment of the invention, the liquid wash fraction is recirculated into the acidic wash step.

By recycling the low phosphorus wash fraction into the acidic wash step, the acid which is present in the low phosphorus wash fraction may advantageously be reused and thus reducing cost as the method requires less use of acid and water.

The biomass digestate may be provided in a number of different ways.

Biomass digestate is the material remaining after the anaerobic digestion of a biodegradable feedstock. Biomass digestate is produced both by acidogenesis and methanogenesis and each has different characteristics. Biomass digestate can be fibrous and consist of structural plant matter including lignin and cellulose. The biomass digestate may also contain minerals and remnants of bacteria.

The biomass digestate may be provided by anaerobically digesting manure and/or deep bedding and/or food waste and/or soft biomass and/or industrial waste products.

It can be advantageous to subject biomasses to a pretreatment step before the anaerobic digestion. The pretreatment can be mechanical and/or thermal and/or chemical and/or biological.

It can be advantageous to subject feedstock to a pretreatment step before the anaerobic digestion. The pretreatment can be mechanical and/or thermal and/or chemical and/or biological.

According to an embodiment of the invention, the biomass digestate is provided by heating a soft biomass in a pretreatment step followed by anaerobic digesting of the soft biomass to obtain the biomass digestate.

An advantage of the pretreatment step is that it makes the soft biomass more accessible for subsequent degradation for example by melting pectin and other waxes away, by mechanically processing the soft biomass or by acidic treatment of the soft biomass, so that e.g. hemicellulose is more accessible for digestion. It may thus also make the resulting biomass digestate more accessible for recovery of nutrients.

The pretreatment step may be performed for less than 7 days, such as less than 6 days, such as less than 5 days, such as less than 4 days, such as less than 3 days, such as less than 2 days, such as less than 1 day.

The pretreatment step may be performed for 2 hours or less.

The pretreatment step may be not heated.

Acid and/or food waste may be added to the soft biomass.

The anaerobic digesting of the pretreated biomass may be performed for less than 10 days.

It should be understood that the biomass digestate may be provided in further ways.

The post-treatment step is a steam explosion, laccase treatment, base treatment, dilute acid treatment, thermal treatment, dissolution in ionic liquids and subsequent precipitation, dissolution in molten salt hydrates, or the organosolv process, or combinations such as steam explosion and laccase.

The posttreatment step may be performed for less than 4 days, such as less than 2 days, such as less than 25 hours, such as less than 24 hours, such as less than 21 hours, such as less than 18 hours, such as less than 15 hours, such as less than 10 hours, such as less than 5 hours, such as less than 2 hours, such as less than 1 hour.

According to an embodiment of the invention the posttreatment temperature is between 40 and 230 degrees Celsius.

The posttreatment temperature may be between 40 and 230 degrees Celsius, such as between 40 and 60 degrees Celsius, such as between 80 and 160 degrees Celsius, such as between 150 and 230 degrees Celsius, such as between 50 and 120 degrees Celsius, such as between 90 and 140 degrees Celsius, such as between 130 and 220 degrees Celsius.

The posttreatment step may be pressurized.

This may for example be the case with steam explosion.

According to an embodiment of the invention, the posttreatment step is performed at a pressure between IMPa (10 bar) and 2 MPa (20 bar).

According to an embodiment of the invention, the posttreatment temperature is between 150 and 230 degrees Celsius, such as between 170 and 210 degrees Celsius, such as between 180 and 200 degrees Celsius.

The posttreatment step may be performed at a pressure between IMPa (10 bar) and 2 MPa (20 bar) and at a temperature between 150 and 230 degrees Celsius, such as between 170 and 210 degrees Celsius, such as between 180 and 200 degrees Celsius.

The posttreatment step may be performed by steam explosion at a temperature between 150 and 230 degrees Celsius, such as between 170 and 210 degrees Celsius, such as between 180 and 200 degrees Celsius.

The posttreatment step may be performed for less than 1 hour.

The posttreatment may be performed by steam explosion and/or laccase treatment.

Laccases (EC.1.10.3.2) are copper-containing oxidase enzymes found in many plants, fungi, and microorganisms. Laccases act on phenols and similar molecules, performing one-electron oxidations, which remain poorly defined.

Laccase treatment may advantageously be performed subsequent to steam explosion. Thereby may a larger amount of biogas be obtained, when the posttreatment is followed an anaerobic digestion. Laccase treatment may be performed e.g. at a temperature around 50 degrees Celsius, such as from 40 to 60 degrees Celsius. When the temperature is too high the activity of laccase is destroyed.

The posttreatment may be performed by bacteria or fungi treatment.

The posttreatment may be performed by base treatment.

The posttreatment temperature may be between 50 and 120 degrees Celsius, such as between 80 and 120 degrees Celsius, such as between 80 and 100 degrees Celsius.

The posttreatment temperature may be performed by base treatment at a temperature between 50 and 120 degrees Celsius, such as between 80 and 120 degrees Celsius, such as between 80 and 100 degrees Celsius.

Ionic liquids (IL) are special solvents that consist of salts with a melting point below 100 °C, high thermal stability and negligible vapor pressure. Ionic liquids can dissolve and/or deconstruct materials that are otherwise considered insoluble in conventional solvents and can thereby increase the biomethane production from otherwise recalcitrant biomass.

The posttreatment may be performed by dissolution in ionic liquids and subsequent precipitation.

The posttreatment step may be performed by dissolution in ionic liquids and subsequent precipitation and at a temperature between 90 and 140 degrees Celsius.

Molten salt hydrates (MSH) are very concentrated metal salt solutions that can dissolve and depolymerize lignocellulose, acting as solvents and/or homogeneous catalysts. Like ionic liquids, MSH have high thermal stability and low volatility, and they have further advantages such as low cost, ease in preparation and low toxicity.

The posttreatment may be performed by dissolution in molten salt hydrates (MSH).

Organosolv is a pulping process for dissolving lignin and hemicellulose in recalcitrant lignocellulose and involves treating the biomass digestate in an organic solvent such as ethanol or a mixture of ethanol and water at temperatures typically between 120 and 220 degree Celsius at elevated pressure. The deconstruction of lignocellulose by an organosolv process opens up the structure, enabling increased biomethane production from cellulose and valuable side streams.

The posttreatment may be performed by organosolv.

The posttreatment step may be performed by dissolution in ionic liquids at a temperature between 90 and 150 degrees Celsius and subsequent precipitation.

The posttreatment may be performed by dilute acid treatment.

The posttreatment may be performed by dilute acid treatment at a temperature between 80 and 160 degrees Celsius.

According to an advantageous embodiment of the invention, furfural and 5-HMF and 2-furoic acid are generated in a combined amount of less than 5% w/w relative to total dry matter i.e. the percentage is referring to weight of total inhibitor relative to total dry matter weight i.e. w/w, and e.g. 10 g inhibitor/kg dry matter is thus equal to 1%. According to an embodiment of the invention, furfural and 5-HMF and 2-furoic acid are generated in a combined amount of less than 5% w/w relative to total dry matter in the post treatment step, such as less than 3% w/w relative to total dry matter in the post treatment step, such as less than 1% w/w relative to total dry matter in the post treatment step, such as less than 0.5% w/w relative to total dry matter in the post treatment step, such as less than 0.06% w/w relative to total dry matter in the post treatment step.

According to an embodiment of the invention, anaerobic digesting of the posttreated solid wash fraction is performed for less than 30 days.

The anaerobic digesting step may be performed by bacteria, such as fermentative bacteria, such as *Ruminococcus albus, Ruminococcus flavefaciens* and/or *Fibrobacter succinogenes.*

The method may further comprise a further separation step after anaerobic digesting the posttreated solid wash fraction to obtain a liquid posttreated biomass digestate fraction and a solid posttreated biomass digestate fraction.

Separation may for example be performed with a screw press, filter press or decanter.

The method may further comprise a drying step of the solid posttreated biomass digestate fraction to obtain a dried posttreated biomass digestate.

By drying the solid posttreated biomass digestate fraction, ammonia may advantageously be removed by evaporation and condensed. The ammonia may then for example be bobbled through sulfuric acid to generate ammonia sulphate, which can be used as e.g. fertilizer. Furthermore, dry lignin may thus be obtained in a purity of more than 30% by weight, such as 35% by weight, such as 40% by weight, since other components such as phosphorus and nitrogen are removed.

The amount of nitrogen in the dried posttreated biomass digestate may be reduced by at least 50%, such as at least 60%, such as at least 70% or such as at least 80% by weight of the nitrogen comprised in the biomass digestate entering the process.

In other words, the amount of nitrogen in the dried posttreated biomass digestate comprises less than 50% of the nitrogen comprised in the biomass digestate or even less than 20%.

At least 50%, such as at least 60%, such as at least 70% or such as at least 80% by weight of the nitrogen comprised in the biomass digestate entering the process may be isolated.

Lignin may be obtained from the method in a purity of at least 30% by weight, such as at least 35% by weight, such as at least 40% by weight.

By subjecting the biomass digestate to the wash step prior to posttreatment and then drying, the purity of lignin may be substantially increased to more than 30% or more than 35% or more than 40% or even higher, since e.g. carbohydrates and nutrients, such as phosphorus and nitrogen are removed, which increases the value of lignin. Lignin may also be obtained from the posttreated biomass digestate by e.g. extraction with an organic solvent such as ethanol or methanol.

According to an embodiment of the invention, lignin is comprised in the posttreated biomass digestate or dried posttreated biomass digestate in an amount of more than 30% by weight, such as in an amount of more than 35% by weight, such as in an amount of more than 40% by weight.

Lignin may be produced substantially free from nutrients and/or carbohydrates, thus obtaining lignin of high quality, which may be used for e.g. bio-pellets or replacement for bitumen and other high value green chemicals.

Biogas may be obtained from at least the anaerobic digesting step of the posttreated solid wash fraction.

Biogas may be obtained from other digestion steps.

Biogas may be only obtained in the anaerobic digestion step.

According to an embodiment of the invention, the method further comprises recirculation of at least a part of a liquid fraction from any separation step to any anaerobic digesting step and/or the pretreatment step.

In this way liquid and bacteria are advantageously reused, thus reducing the cost of the method.

### FIGURES

Figure 1 illustrates a method for processing biomass digestate according to an embodiment of the invention.
Figure 2 illustrates a method for processing biomass according to an embodiment of the invention.
Figure 3 illustrates removal of phosphorus from a solid biomass digestate fraction within 1 hour as a result of pH in the acidic wash step.
Figure 4 illustrates removal of phosphorus from a solid biomass digestate fraction as a result of pH, temperature and time.
Figure 5 illustrates removal of phosphorus from liquid wash fractions.
Figure 6 illustrates the amount of additional methane produced as a result of different post-treatments or no posttreatment.
Figure 7 illustrates the amount of additional methane produced as a result of different post-treatments relative to no post-treatment.
Figure 8 illustrates removal of phosphorus from a solid biomass digestate fraction after incubation at 50 °C during 24 h at various starting pH values.
Figure 9 illustrates removal of phosphorus from a solid biomass digestate fraction as a function of time at four different starting pH values at 50 °C.
Figure 10 illustrates removal of phosphorus from a solid biomass digestate fraction after 24 h at different starting pH values at 50 °C and 80 °C.

### DETAILED DESCRIPTION

As used herein, the term "non-pressurized" is intended to mean ambient pressure, which at average sea level is a pressure around 1 bar = 101 kPa.

As used herein, the term "pressurized" is intended to mean a pressure significantly higher than ambient pressure. Usually this may be around 1-2 MPa (10-20 bar), especially in the field of biogas production.

As used herein "biomass digestate" is intended to mean a product of anaerobic digestion AD of soft biomass and represents the digested material, which is removed from the AD reactor (digestor) after recovery of biogas. Digestate is normally liquid. As used herein, the term "soft biomass" is intended to mean non-wood lignocellulosic biomass, comprising cellulose, hemicellulose and lignin. Soft biomass may e.g. be wheat straw, corn stover, rice straw, grass, and bagasse.

The term "soft biomass" may mean cellulosic and herbaceous types of biomass, such as wheat straw, corn stover, rice straw, grass, and bagasse.

As used herein, the term "dry matter" is intended to mean the residual when water is evaporated.

Lignocellulosic biomass usually comprises crystalline cellulose fibrils intercalated within a loosely organized matrix of hemicellulose and sealed within an environment rich in hydrophobic lignin. While cellulose itself comprises long, straight chain polymers of D-glucose, hemicellulose is a heterogeneous mixture of short, branched-chain carbohydrates including monomers of all the 5-carbon aldopentoses (C5 sugars) as well as some 6- carbon (C6) sugars including glucose and mannose. Lignin is a highly heterogeneous polymer, lacking any particular primary structure, and comprising hydrophobic phenylpropanoid monomers. Suitable lignocellulosic biomass typically comprises cellulose in amounts between 20 and 50 % of dry mass prior to pretreatment, lignin in amounts between 10 and 40 % of dry mass prior to pretreatment, and hemicellulose in amounts between 15 and 40%.

As used herein, the term "liquid fraction" is intended to mean the fraction having the lowest dry matter after a separation step. The amount of suspended solids is normally around 4% per weight, but typically varies from 0 to less than 10%.

As used herein, the term "solid fraction" intended to mean the fraction having the highest dry matter after a separation step. The amount of suspended solids is normally around 20-25% per weight, but may vary from 10-95%.

As used herein, the term "anaerobic digestion" is intended to mean a collection of processes by which microorganisms break down break down biodegradable material in the absence of oxygen. Anaerobic digestion usually begins with bacterial hydrolysis of the input materials. Insoluble organic polymers, such as carbohydrates, are broken down to soluble derivatives that become available for other bacteria. Acidogenic bacteria may then convert the sugars and amino acids into carbon dioxide, hydrogen, ammonia and organic acids. These bacteria may convert these resulting organic acids into acetic acid, along with additional ammonia, hydrogen, and carbon dioxide. Finally, methanogens may convert these products to methane and carbon dioxide. Anaerobic digestion is widely used as a source of renewable energy. The process produces a biogas, comprising mostly methane and carbon dioxide.

As used herein, the term "pretreatment" is intended to mean a treatment of a biomass prior to a first anaerobic digestion step.

As used herein, the term "posttreatment" is intended to mean a treatment of a biomass digestate subsequent to the acidic wash step and prior to anaerobic digestion of the posttreated solid wash fraction, wherein the posttreatment is selected from the list consisting of steam explosion, laccase treatment, base treatment, dilute acid treatment, thermal treatment, dissolution in ionic liquids and subsequent precipitation, dissolution in molten salt hydrates, or an organosolv process, or combinations thereof.

The posttreatment increases the yield of biogas in the subsequent anaerobic digestion (of the posttreated solid wash fraction). A purpose of the posttreatment is thus to increase the digestability of the solid wash fraction prior to anaerobic digestion. Thus, the posttreatment step could be considered a digestion preparation step, a biomass preparation step or simply a preparation step. Alternatively, the posttreatment step could be considered treatment for maturing the biomass for digestion i.e. a biomass maturation step.

The aim of posttreatment step is to loosen, degrade or dissolve the lignin layer that protects the cellulose and hemicellulose and/or reduce the crystallinity of cellulose in order to make the biomass more accessible for digestion.

The posttreatment step includes very diverse processes such as biological-, non-biological, chemical and physiochemical processes. Hence, post-treatment step is selected from the list consisting of steam explosion, laccase treatment, base treatment, dilute acid treatment, thermal treatment, dissolution in ionic liquids and subsequent precipitation, dissolution in molten salt hydrates, or the organosolv process, or combinations such as steam explosion and laccase.

As used herein nitrogen (N) as is intended to mean the sum of organic bound nitrogen, such as the nitrogen in amines, and inorganic bound nitrogen, such as e.g. NH₃ and NH₄⁺.

As used herein phosphorus (P) is intended to mean the sum of organic bound P and inorganic bound P, such as e.g. PO₄ ions (e.g. PO₄⁻).

As used herein "nutrients" is intended to mean macronutrients for plants, such as nitrogen (N), phosphorus (P), potassium (K), calcium (Ca) and sulfur (S).

As used herein "strong acid" is meant to be an acid having a pKa below 3.

As used herein "weak acid" is meant to be an acid having a pKa above 3.

As used herein, the singular forms "a", "an" and "the" include plural referents unless the context clearly dictates otherwise.

As used herein, "at least one" is intended to mean one or more, i.e. 1, 2, 3, 4, 5, 6, 7, 8, 9, 10, etc.

As used herein, the term "biogas" is intended to mean methane gas and carbondioxide gas obtained from degradation of biological material, such as biomass.

As used herein, the term "inoculum" is intended to mean organic material containing bacteria, such as digested (degassed) biomass from an existing biogas facility or animal waste.

As used herein, the term "dry matter" is intended to mean the residual when water is evaporated.

As used herein, the term volatile solids (VS) shall mean the organic part of dry matter. Separation may for example be performed with a screw press, filter press or decanter.

### Abbreviations

VS = volatile solids
5-HMF = 5-(hydroxymethyl)furfural
TS = total solids

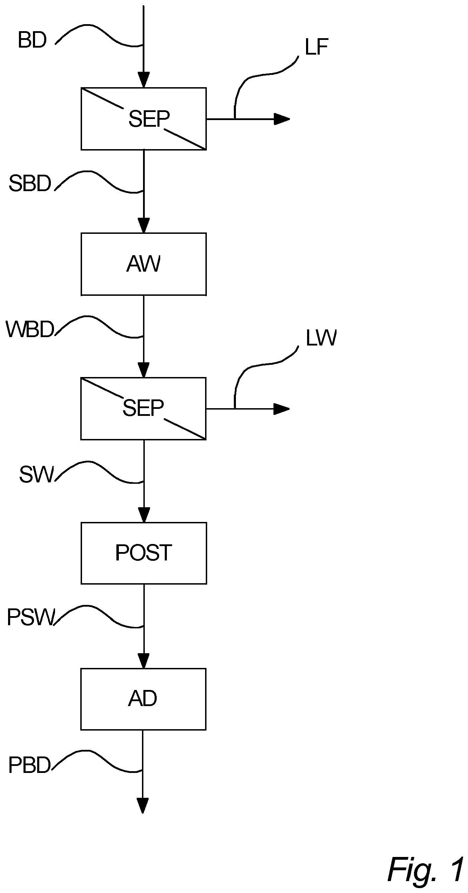

Figure 1 shows a method for processing biomass digestate according to an embodiment of the invention.

Biomass digestate (which may be pretreated) is separated into a solid biomass digestate fraction, such as a fiber fraction, and a liquid fraction. The liquid fraction contains a lower phosphorus concentration compared to the digestate and can be used as fertilizer.

The solid fraction is mixed with acid in a wash reactor in which phosphorus will be dissolved from fibers into the liquid phase. Afterwards the slurry is separated into a solid washed fiber fraction and a liquid wash fraction.

The solid wash fraction (fiber fraction) is posttreated to increase the digestibility of the fiber. The posttreated solid wash fraction is transferred to another biogas reactor and a second anaerobic digestion process is performed to produce more biogas.

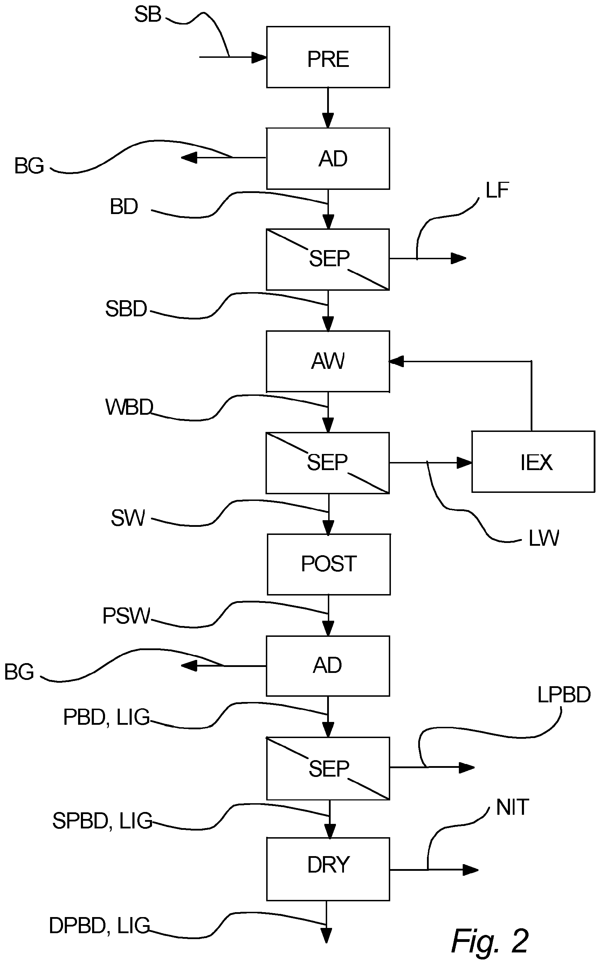

Referring to figure 2, a method for processing biomass digestate according to an embodiment of the invention is disclosed.

Biomass digestate (which may be pretreated) is separated into a solid biomass digestate fraction, such as a fiber fraction, and a liquid fraction. The liquid fraction contains a lower phosphorus concentration compared to the digestate and can be used as fertilizer. The solid fraction is mixed with acid in a wash reactor in which the phosphorus will be dissolved from fibers into the liquid phase. Afterwards the slurry is separated into a solid washed fiber fraction and a liquid wash fraction. The liquid wash fraction may e.g. be transferred to an ion exchange column, in which the phosphorus binds to the resin in the ion exchange column. The ion exchanged liquid wash fraction contains a very low amount of phosphorus and may advantageously be transferred back to the wash reactor.

The phosphorus bound to the resin may be released by e.g. increasing the pH with NaOH, and thereby producing Na₃PO₄. The solid wash fraction (fiber fraction) is post treated to increase the digestibility of the fiber. The posttreated solid wash fraction is transferred to another biogas reactor to produce more biogas. After anaerobic digestion in a biogas process, the slurry may be pressed i.e. separated into a posttreated biomass digestate liquid fraction and a posttreated biomass digestate solid fraction, which may be referred to as a lignin fraction, since this fraction comprises high amounts of lignin. The posttreated biomass digestate liquid fraction may be transferred back to the biogas reactor. The lignin fraction can then be dried. In the dryer, the ammonium will evaporate and the lignin will be dry and of a high purity.

### EXAMPLES

### Example 1: Washing phosphorus out of fiber with sulphuric acid and hydrochloric acid

This example describes how to wash the phosphorus out of biomass digestate, such as a solid biomass digestate fraction, with acid. A fixed amount of fiber i.e. solid biomass digestate has been mixed with different types of acid with varying pH.

### Materials and methods:

- Fiber, produced from separating digestate from a biogas plant into a solid biomass digestate fraction
- Water
- Sulphuric acid (H₂SO₄)
- Hydrochloric acid (HCl)

Four washes were made to test sulphuric acid and hydrochloric acid at different pH. 900 g of water was mixed with sulphuric acid and hydrochloric acid, respectively. Two samples were mixed to a pH of 1 and two samples to a pH of 3. 90 g of fiber was added to each of the four samples and mixed. After 1 h at 25 °C, fiber and wash water were separated and phosphorus was measured the liquid wash fraction as well as in an untreated fiber sample, in order to find out how much phosphorus was separated into the liquid wash fraction.

### Results:

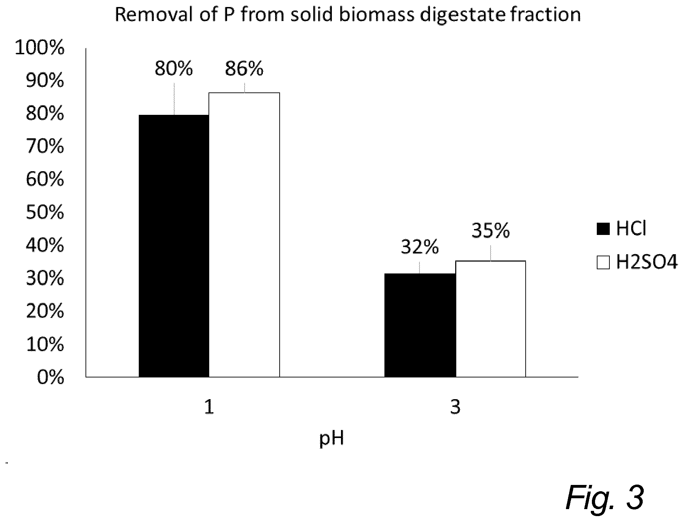

The results show that pH has a large impact on the dilution of phosphorus into the wash-liquid within the first hour. The lower the pH, the more phosphorus is removed from the fiber, as seen on Figure 3.

### Example 2: Washing phosphorus out of the solid biomass digestate fraction with oxalic acid varying pH, incubation temperature and -time.

This example describes how to wash the phosphorus out of solid biomass digestate fraction with oxalic acid. A fixed amount of solid biomass digestate fraction has been mixed with oxalic acid at varying pH, incubation temperature and incubation time.

### Materials and methods:

- Solid biomass digestate fraction (fiber fraction, produced from separating digestate from a biogas plant)
- Water
- Oxalic acid (C₂H₂O₄)

A Design of Experiments (DOE) software "MODDE Go" (version 12.1.0.5491, Mar 23 2018) was used to design the experiment and analyze the results. Washes were made with oxalic acid in which three factors were varied; pH of solution, incubation temperature and incubation time. The pH were 0.79, 0.92 and 1.26, the incubation temperatures were 40 °C, 70 °C and 100 °C, and the incubation times were 10 min, 2 hours, and 3 hours and 50 min. For each wash, 900 g of water was mixed with oxalic acid to the desired pH and 90 g of solid biomass digestate fraction was added and mixed. The factors were combined as shown in Table 1.

**Table 1: Scheme of how the factors are combined in the different samples.**

| **Sample** | **pH of acid solution** | **Incubation temperature (°C)** | **Incubation time** |
|---|---|---|---|
| 1 | 1.26 | 40 | 10min |
| 2 | 1.26 | 100 | 10min |
| 3 | 1.26 | 70 | 2 hours |
| 4 | 1.26 | 40 | 3 hours 50 min |
| 5 | 1.26 | 100 | 3 hours 50 min |
| 6 | 0.79 | 40 | 10min |
| 7 | 0.79 | 100 | 10min |
| 8 | 0.79 | 70 | 2 hours |
| 9 | 0.79 | 40 | 3 hours 50 min |
| 10 | 0.79 | 100 | 3 hours 50 min |
| 11 | 0.92 | 40 | 10min |
| 12 | 0.92 | 100 | 10min |
| 13 | 0.92 | 70 | 2 hours |
| 14 | 0.92 | 70 | 2 hours |
| 15 | 0.92 | 70 | 2 hours |
| 16 | 0.92 | 40 | 3 hours 50 min |
| 17 | 0.92 | 100 | 3 hours 50 min |

After each wash, the washed biomass digestate was separated into a solid fraction and a liquid wash fraction and phosphorus was measured on the liquid wash fraction as well as on an untreated solid biomass digestate fraction sample, in order to find out how much phosphorus was diluted (i.e. removed from the solid fraction) in the liquid wash fraction.

### Results:

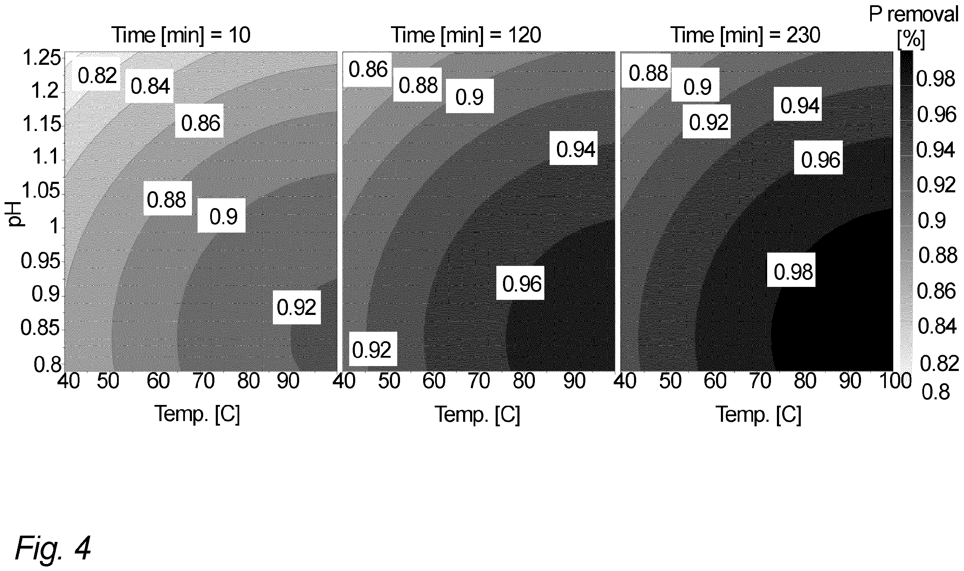

As the experiment was designed with use of a statistical design, a function is calculated on basis of all the data points by the program MODDE Go, and the result of the experiments can thus be seen in Figure 4. Selected raw data points are shown in table 2a-2b, however one should not rely entirely on a single value but instead look at the function calculated on basis of all the data points as shown in Figure 4. Sample 13, 14 and 15 are replicates, and from these the uncertainty of the results is found to be +/-1%. The function has a high R2 value of 88% which is the percent of the variation of the response explained by the model.

The results show that it is possible to remove 99% of the phosphorus from the solid biomass digestate fraction. From figure 4, one can see that the lower the pH is and/or the higher the temperature and/or the longer the washing time, the more phosphorus is removed from the solid biomass digestate fraction (and thus present in the liquid wash fraction).

**Table 2a: Raw data points used to calculate the function illustrated in Figure 4. Amount of phosphorus removed from the solid biomass digestate fraction by washing with an aqueous acidic solution.**

| **Sample** | 1 | 2 | 3 | 4 | 5 | 6 | 7 | 8 | 9 | 10 |
|---|---|---|---|---|---|---|---|---|---|---|
| **P removal** | 75% | 85% | 90% | 89% | 94% | 89% | 95% | 96% | 94% | 96% |

**Table 2b: Raw data points used to calculate the function illustrated in Figure 4. Amount of phosphorus removed from the solid biomass digestate fraction by washing with an aqueous acidic solution.**

| **Sample** | 11 | 12 | 13 | 14 | 15 | 16 | 17 |
|---|---|---|---|---|---|---|---|
| **P removal** | 85% | 91% | 96% | 94% | 95% | 91% | 99% |

Another series of experiments were conducted. The treatment was performed at 50 °C for 24 hours. The starting pH was varied from 2 to 8.7. At these conditions the pH changes during the treatment, but the reported values are starting values, i.e. measured pH immediately after mixing of water, oxalic acid and fibers. The procedure was the same as described above.

### Results:

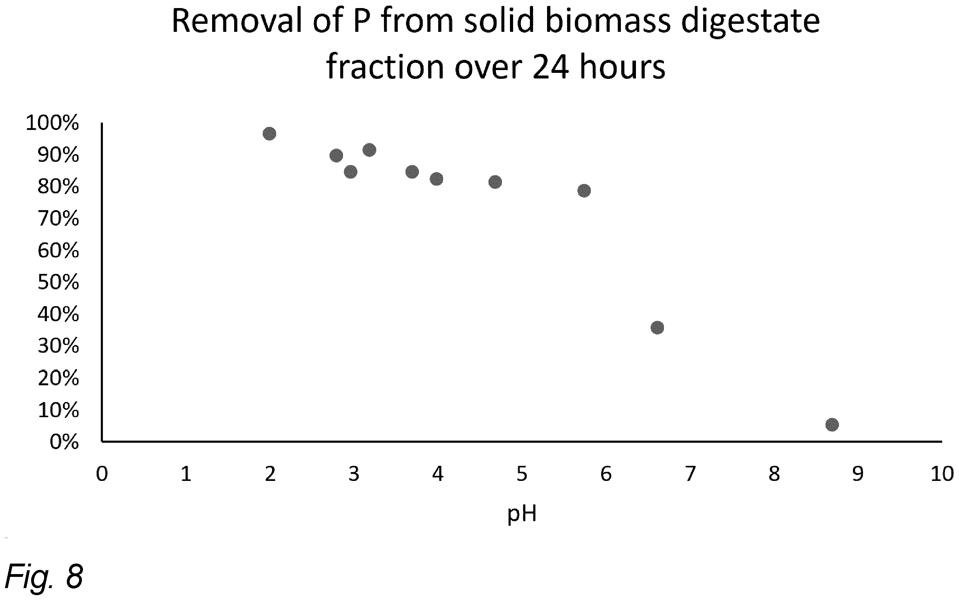

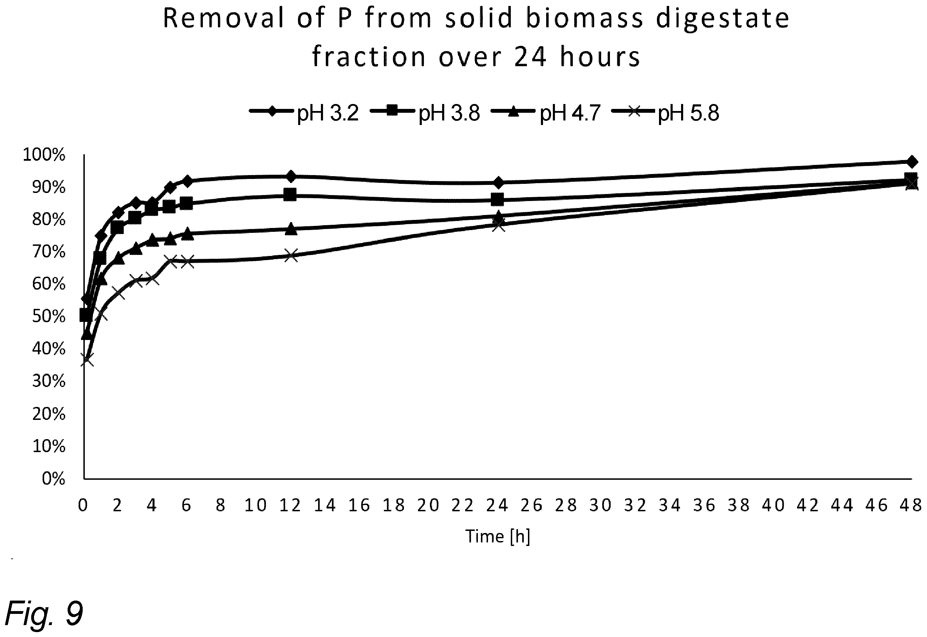

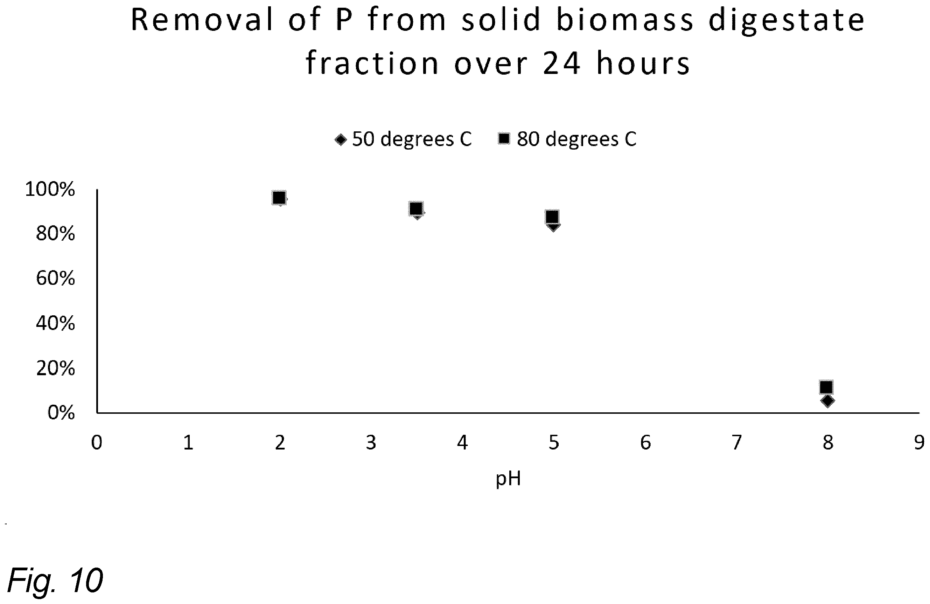

Figure 8 shows the percentage of phosphorus removed from the solid biomass digestate fraction after incubation at 50 °C during 24 h at various starting pH values. It is seen that the phosphorus can be removed efficiently up until at least pH 5.75 under these conditions. Figure 10 shows a comparison of selected experiments from Figure 8 and corresponding experiments run at 80 °C. It can be concluded that the temperature plays a minor role at the conditions investigated. The removal of phosphorus was followed as a function of time for four different starting pH values. The results are plotted in Figure 9 and show that most of the phosphorus is washed out during the first six hours for the lower pH values of 3.2 and 3.8. Incubation times of up to 48 hours are needed to reach the same level of phosphorus removal for pH values of 4.7 and 5.8.

### Example 3: Removing phosphorus from the wash-liquid fraction

This example describes how to remove the phosphorus by means of ion exchange from the liquid wash fraction obtained from the acidic wash step.

An acid wash step has been performed prior. The wash was made by mixing 900 g of water with oxalic acid to a pH of 1 and adding 90 g of fiber (solid biomass digestate fraction produced from separating digestate from a biogas plant) with an incubation temperature of 25 °C and an incubation time of 15 hours. Afterwards the washed biomass digestate was separated into a solid wash fraction (fiber fraction) and liquid wash fraction, and the liquid wash fraction was treated.

### Materials and methods:

- Liquid wash fraction obtained from acid wash of solid biomass digestate fraction (type of wash is described above)
- Resin 1 (Lewatit FO 36, Lanxess)
- Resin 2 (Amberlite MB 3, Merck)

20 g of liquid wash fraction was mixed with 20 g of resin 1, and 20 g of liquid wash fraction was mixed with 20 g of resin 2 in blue cap bottles. The bottles were incubated at 50 °C for an hour with stirring, and afterwards they were centrifuged to separate the resins from the liquid wash fraction. Phosphorus was measured in the liquid wash fraction without subjecting it to ion exchange, and the two ion exchange treated liquid wash fractions to find out how much phosphorus the two resins are able to remove.

### Results:

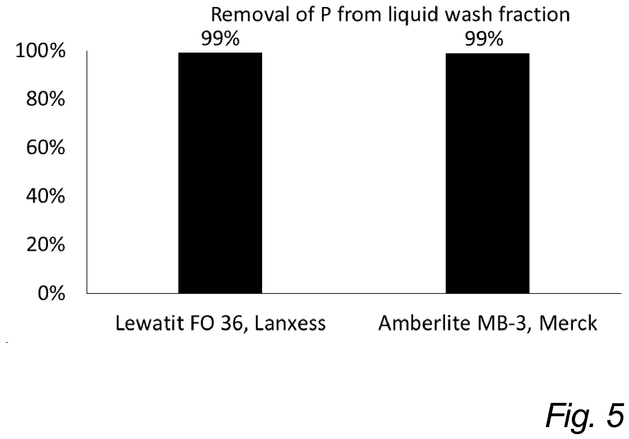

By using ion exchange, it is possible to remove 99% of the phosphorus in the liquid wash fraction obtained from acid wash of solid biomass digestate fraction, as seen in Figure 5. Furthermore, both resins tested can be used for phosphorus removal.

### Example 4: Extra methane as a result of post-treatment

This example describes how to produce extra biogas from biomass digestate as a result of different types of post-treatment of solid wash fractions.

Prior to the post-treatments, an acidic wash of solid biomass digestate fractions was performed. The acidic wash was made by mixing 891 g of water with 9 g of oxalic acid so it had a concentration of 0.1 mol/L and adding 90 g of solid biomass digestate (produced from separating digestate from a biogas plant) with an incubation temperature of 50 °C and an incubation time of 3 hours. Afterwards the washed biomass digestate was separated into a solid wash fraction and a liquid wash fraction, and the solid wash fraction was post-treated.

### Materials and methods:

- Solid wash fraction from acid wash (type of wash is described above)
- Water
- Citric acid
- Sodium hydroxide (NaOH)
- Laccase
- Inoculum (reactor liquid from a biogas plant)

Five different samples for posttreatment and subsequent anaerobic digestion were prepared. The first sample was just solid wash fraction from acid wash with no posttreatment. The second sample was solid wash fraction from acid wash, which was post-treated with steam explosion. The third sample was solid wash fraction from acid wash, which was post-treated with laccase. The fourth sample was solid wash fraction from acid wash, which was post-treated with steam explosion and afterwards laccase. The fifth sample was solid wash fraction from acid wash, which was posttreated with sodium hydroxide. The steam explosion was performed by mixing 40 g of solid wash fraction and 160 g of water in a Parr Reactor, which was run for 15 min at 180 °C. The laccase treatment was performed by mixing 8 g of solid wash fraction with 90 g of a 0.1 M citric acid buffer adjusted to pH 5 with NaOH. 0.6 g laccase/g TS solid wash fraction was added and the sample was stirred for 24 hours at 250 rpm in a shaking incubator at 45 °C. The base treatment was performed by mixing 15 g of solid wash fraction with 90 g of water and adding NaOH to reach a pH of 12. The sample was heated to 100 °C for 20 hours. After each post-treatment, the five samples were adjusted to a pH of 8 with NaOH/HCl and mixed with inoculum and anaerobic digested for 15 days at a temperature of 52 °C.

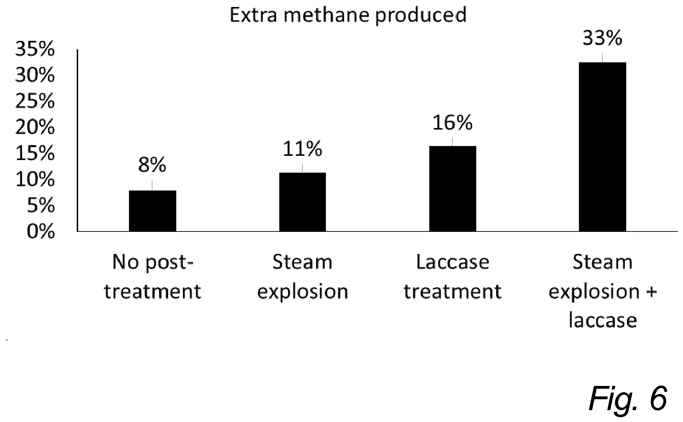

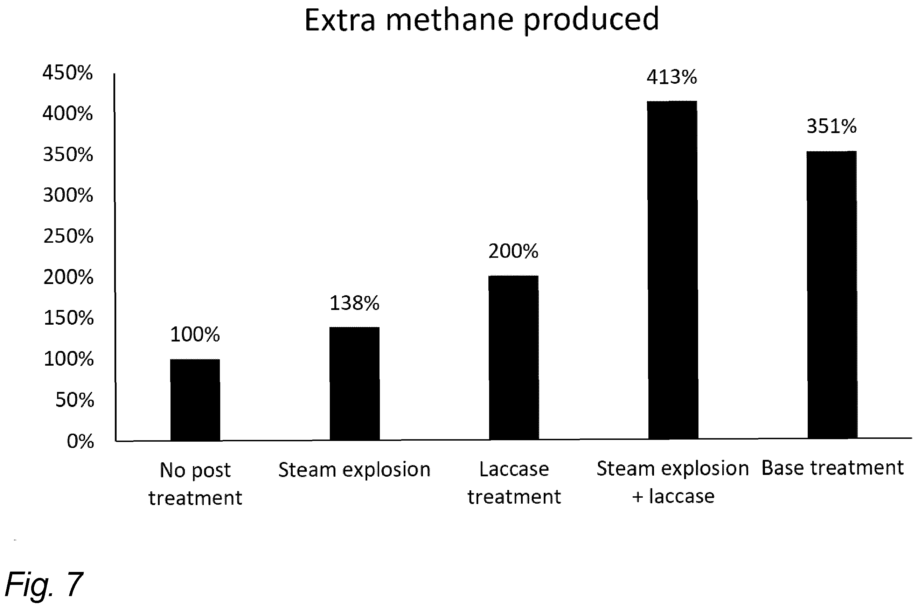

Results: Additional biogas was obtained for all samples as depicted in figure 6 and 7. The results depicted in figure 6 illustrates the additional methane produced by processing digestate including a posttreatment step or not.

The results depicted in figure 7 are normalized to the result obtained when no post-treatment was performed. Steam explosion resulted in 138% extra methane, laccase treatment resulted in 200% extra methane. Combining the two methods by posttreating the solid wash fraction with steam explosion followed by laccase treatment resulted in a surprising 413 % extra methane . Base treatment resulted in 351% extra methane.

Base treatment was further explored with experiments run at different temperatures varying from 50 degrees Celsius to 120 degrees Celsius. A satisfying result was obtained for all tested temperatures in between 50 degrees Celsius and 120 degrees Celsius.

### Example 5: Lignin product description

The posttreated biomass digestate from Example 4 were dried and the resulting dried posttreated biomass digestate was a lignin fraction comprising the following:

| | |
|---|---|
| Lignin: | 36 % |
| Water content: | 12% |
| Ash: | 4.5% |
| Chlorine: | 0.013% |
| Sulphur: | 0.4% |
| Calorific value: | 18. MJ/kg |

### Example 6.

Biomass digestate may for example advantageously be provided by subjecting a soft biomass to heating in a non-pressurized pretreatment step at a pretreatment temperature between 65 to 100 degrees Celsius at ambient pressure. The heating may for example be performed in a tank or container with or without stirring. The retention time in the pretreatment step is usually short, such as less than one hour or less than 30 minutes, but it may also be longer if this may be convenient. The pretreated biomass may then be conveyed into a further tank to be anaerobic digested to provide a biomass digestate. The anaerobic digestion may be performed for example by the addition of bacteria. The anaerobic digestion is usually performed at a pH around neutral, such as for example between pH 6-8. An important degradation product, which may be obtained from the anaerobic digestion step is biogas, comprising methane and/or carbon dioxide.

### Example 7: Removing phosphorus from the liquid wash fraction using base precipitation

This example describes how to remove the phosphorus by addition of base from the liquid wash fraction obtained from the acidic wash step.

An acid wash step has been performed prior. The wash was made by mixing 5400 g of water with 15 g of acetic acid and adding 540 g of fiber (solid biomass digestate fraction produced from separating digestate from a biogas plant) with an incubation temperature of 50 °C and an incubation time of 24 hours. Afterwards the washed biomass digestate was separated into a solid wash fraction (fiber fraction) and liquid wash fraction, and the liquid wash fraction was treated.

### Materials and methods:

- Liquid wash fraction obtained from acid wash of solid biomass digestate fraction (type of wash is described above)
- Calcium hydroxide, Ca(OH)₂ (>96%, Chemsolute)
- Sodium hydroxide, NaOH (99.5%, Chemsolute)

The liquid wash fraction was divided into 4 samples as shown in the table. The content of phosphorus was measured using inductively coupled plasma method optical emission spectrometry (ICP-OES). The stated amounts (in weight percent of the liquid wash fraction) of solid calcium hydroxide or sodium hydroxide were added to the samples and stirred for 1 h at ambient temperature. The pH reached 8.9 in sample 1-3 and 8.7 in sample 4. The precipitation was removed by centrifugation and the phosphorus concentration in the base-treated liquid wash fraction was measured again using ICP.

### Results:

It is possible to remove at least 98% of the phosphorus from the liquid wash fraction by increasing the pH value using a base such as for example calcium hydroxide or sodium hydroxide. Addition of more base would likely further increase the pH value as well as improve the phosphorus removal.

**Table 3: Phosphorus removal using base precipitation.**

| **Sample nr.** | **m(LW) [g]** | **P conc. [mg/L] before base addition** | **m(Ca(OH)₂) [g]** | **m (NaOH) [g]** | **wt% base** | **P conc. [mg/L] after base addition** | **P conc. reduction** |
|---|---|---|---|---|---|---|---|
| 1 | 1000 | 125 | 0.48 | 0.00 | 0.048% | 2.7 | 98% |
| 2 | 1000 | 125 | 0.48 | 0.00 | 0.048% | 2.7 | 98% |
| 3 | 1001 | 118 | 0.45 | 0.00 | 0.045% | 2.6 | 98% |
| 4 | 1000 | 118 | 0.00 | 034 | 0.034% | 4.0 | 97% |

### FIGURE REFERENCES

DRY. Drying step
BD. Biomass digestate
SEP. Separation step
LF. Liquid fraction
SBD. Solid biomass digestate fraction
AW. Acidic wash step
WBD. Washed biomass digestate,
LW. Liquid wash fraction
SW. Solid wash fraction
POST. Posttreating
PSW. Posttreated solid wash fraction
PRE. pretreating
AD. Anaerobic digesting
PBD. Posttreated biomass digestate
IEX. ion exchange
LPBD. Liquid posttreated biomass digestate fraction
SPBD. Solid posttreated biomass digestate fraction
DPBD. Dried posttreated biomass digestate
LIG. Lignin
DRY. Drying step
BG. Biogas
SB. Soft Biomass
NIT. Nitrogen

## Claims

1. A method for processing biomass digestate, the method comprising the steps of
- providing a biomass digestate (BD),
- subj ecting the biomass digestate to a separation step (SEP) into a liquid fraction (LF) and a solid biomass digestate fraction (SBD),
- subjecting the solid biomass digestate fraction (SBD) to an acidic wash step (AW) at a pressure below 200 kPa (2 bar) to obtain a washed biomass digestate (WBD),
- separating (SEP) the washed biomass digestate (WBD) into a liquid wash fraction (LW) and a solid wash fraction (SW),
- posttreating (POST) the solid wash fraction (SW) to obtain a posttreated solid wash fraction (PSW) and
- anaerobic digesting (AD) the posttreated solid wash fraction (PSW) to obtain a posttreated biomass digestate (PBD),
wherein the amount of phosphorus in the solid wash fraction (SW) is reduced compared to amount of phosphorus in the solid biomass digestate fraction (SBD), wherein the posttreatment is selected from the list consisting of steam explosion, laccase treatment, base treatment, dilute acid treatment, thermal treatment, dissolution in ionic liquids and subsequent precipitation, dissolution in molten salt hydrates, or an organosolv process, or combinations thereof.

2. The method according to claim 1, wherein the acidic wash step (AW) is non-pressurized.

3. The method according to claim 1 or 2, wherein the amount of phosphorus in the solid wash fraction (SW) is less than 10% by weight of the phosphorus in the biomass digestate (BD).

4. The method according to any of the preceding claims, wherein the pH in the acidic wash step (AW) is lower than 6.5, such as lower than 6, such as lower than 4.

5. The method according to any of the preceding claims, wherein the temperature in the acidic wash step (AW) is between 50 to 100 degrees Celsius.

6. The method according to any of the preceding claims, wherein the acid in the acidic wash step (AW) is a strong acid having a pKa below 3 or a weak acid having a pKa above 3.

7. The method according to any of the preceding claims, wherein the acid in the acidic wash step (AW) is an organic acid.

8. The method according to any of the preceding claims, wherein the amount of phosphorus in the liquid wash fraction (LW) is reduced to obtain a low phosphorus liquid fraction.

9. The method according to any of the preceding claims, wherein the amount of phosphorus in the liquid wash fraction (LW) is reduced by subjecting the liquid wash fraction (LW) to ion exchange (IEX).

10. The method according to any of claims 1-8, wherein the amount of phosphorus in the liquid wash fraction (LW) is reduced by precipitation with a base.

11. The method according to any of the preceding claims, wherein the liquid wash fraction (LW) is recirculated into the acidic wash step (AW).

12. The method according to any of the preceding claims, wherein the biomass digestate (BD) is provided by heating a soft biomass (SB) in a pretreatment step (PRE) followed by anaerobic digesting (AD) of the soft biomass (SB) to obtain the biomass digestate (BD).

13. The method according to any of the preceding claims, wherein the posttreatment (POST) temperature is between between 40 and 230 degrees Celsius, such as between 40 and 60 degrees Celsius, such as between 80 and 160 degrees Celsius, such as between 150 and 230 degrees Celsius, such as between 50 and 120 degrees Celsius, such as between 90 and 140 degrees Celsius, such as between 130 and 220 degrees Celsius.

14. The method according to any of the preceding claims, wherein the posttreatment (POST) step is performed at a pressure between IMPa (10 bar) and 2 MPa (20 bar).

15. The method according to any of the preceding claims, wherein the posttreatment (POST) temperature is between 150 and 230 degrees Celsius, such as between 170 and 210 degrees Celsius, such as between 180 and 200 degrees Celsius.

16. The method according to any of the preceding claims, wherein furfural and 5-HMF and 2-furoic acid are generated in a combined amount of less than 5% w/w relative to total dry matter in the post treatment step (POST), such as less than 3% w/w relative to total dry matter in the post treatment step (POST), such as less than 1% w/w relative to total dry matter in the post treatment step (POST), such as less than 0.5% w/w relative to total dry matter in the post treatment step (POST), such as less than 0.06% w/w relative to total dry matter in the post treatment step (POST).

17. The method according to any of the preceding claims, wherein anaerobic digesting (AD) of the posttreated solid wash fraction (PSW) is performed for less than 30 days.

18. The method according to any of the preceding claims, wherein lignin (LIG) is comprised in the posttreated biomass digestate (PBD) or dried posttreated biomass digestate (DPBD) in an amount of more than 30% by weight, such as in an amount of more than 35% by weight, such as in an amount of more than 40% by weight.

19. The method according to any of the preceding claims, wherein the method further comprises recirculation of at least a part of a liquid fraction from any separation step (SEP) to any anaerobic digesting step (AD) and/or the pretreatment step (PRE).

## Patentansprüche

1. Verfahren zum Verarbeiten eines Biomassengärrests, wobei das Verfahren die folgenden Schritte umfasst
- Bereitstellung eines Biomassengärrests (BD),
- Unterziehen des Biomassengärrests einem Trennschritt (SEP) in eine flüssige Fraktion (LF) und eine feste Biomassengärrestfraktion (SBD),
- Unterziehen der festen Biomassengärrestfraktion (SBD) einem Säurewaschschritt (AW) bei einem Druck unter 200 kPa (2 bar), um einen gewaschenen Biomassengärrest (WBD) zu erhalten,
- Trennen (SEP) des gewaschenen Biomassengärrestes (WBD) in eine flüssige Waschfraktion (LW) und eine feste Waschfraktion (SW),
- Nachbehandeln (POST) der festen Waschfraktion (SW), um eine nachbehandelte feste Waschfraktion (PSW) zu erhalten, und
- anaerobes Vergären (AD) der nachbehandelten festen Waschfraktion (PSW), um einen nachbehandelten Biomassengärrest (PBD) zu erhalten,
wobei die Phosphormenge in der festen Waschfraktion (SW) im Vergleich zur Phosphormenge in der festen Biomassengärrestfraktion (SBD) reduziert ist,
wobei die Nachbehandlung aus der Liste ausgewählt ist, die aus Dampfexplosion, Laccasebehandlung, Basenbehandlung, Dünnsäurebehandlung, thermischer Behandlung, Auflösen in ionischen Flüssigkeiten und anschließendes Ausfällen, Auflösen in geschmolzenen Salzhydraten oder einem Organosolv-Verfahren oder Kombinationen davon besteht.

2. Verfahren nach Anspruch 1, wobei der Säurewaschschritt (AW) drucklos ist.

3. Verfahren nach Anspruch 1 oder 2, wobei die Phosphormenge in der festen Waschfraktion (SW) weniger als 10 Gew.-% des Phosphors in dem Biomassengärrest (BD) beträgt.

4. Verfahren nach einem der vorhergehenden Ansprüche, wobei der pH-Wert im Säurewaschschritt (AW) niedriger als 6,5, wie etwa niedriger als 6, wie etwa niedriger als 4, ist.

5. Verfahren nach einem der vorhergehenden Ansprüche, wobei die Temperatur im Säurewaschschritt (AW) zwischen 50 und 100 Grad Celsius liegt.

6. Verfahren nach einem der vorhergehenden Ansprüche, wobei die Säure im Säurewaschschritt (AW) eine starke Säure mit einem pKa unter 3 oder eine schwache Säure mit einem pKa über 3 ist.

7. Verfahren nach einem der vorhergehenden Ansprüche, wobei die Säure im Säurewaschschritt (AW) eine organische Säure ist.

8. Verfahren nach einem der vorhergehenden Ansprüche, wobei die Phosphormenge in der flüssigen Waschfraktion (LW) reduziert wird, um eine flüssige Fraktion mit niedrigem Phosphorgehalt zu erhalten.

9. Verfahren nach einem der vorhergehenden Ansprüche, wobei die Phosphormenge in der flüssigen Waschfraktion (LW) reduziert wird, indem die flüssige Waschfraktion (LW) einem Ionenaustausch (IEX) unterzogen wird.

10. Verfahren nach einem der Ansprüche 1-8, wobei die Phosphormenge in der flüssigen Waschfraktion (LW) durch Fällung mit einer Base reduziert wird.

11. Verfahren nach einem der vorhergehenden Ansprüche, wobei die flüssige Waschfraktion (LW) in den Säurewaschschritt (AW) zurückgeführt wird.

12. Verfahren nach einem der vorhergehenden Ansprüche, wobei der Biomassengärrest (BD) durch Erhitzen einer weichen Biomasse (SB) in einem Vorbehandlungsschritt (PRE) gefolgt von einer anaeroben Vergärung (AD) der weichen Biomasse (SB), um den Biomassengärrest (BD) zu erhalten, bereitgestellt wird.

13. Verfahren nach einem der vorhergehenden Ansprüche, wobei die Temperatur der Nachbehandlung (POST) zwischen 40 und 230 Grad Celsius beträgt, wie etwa zwischen 40 und 60 Grad Celsius, wie etwa zwischen 80 und 160 Grad Celsius, wie etwa zwischen 150 und 230 Grad Celsius, wie etwa zwischen 50 und 120 Grad Celsius, wie etwa zwischen 90 und 140 Grad Celsius, wie etwa zwischen 130 und 220 Grad Celsius.

14. Verfahren nach einem der vorhergehenden Ansprüche, wobei der Nachbehandlungs(POST)-Schritt bei einem Druck zwischen 1 MPa (10 bar) und 2 MPa (20 bar) durchgeführt wird.

15. Verfahren nach einem der vorhergehenden Ansprüche, wobei die Temperatur der Nachbehandlung (POST) zwischen 150 und 230 Grad Celsius liegt, wie etwa zwischen 170 und 210 Grad Celsius, wie etwa zwischen 180 und 200 Grad Celsius.

16. Verfahren nach einem der vorhergehenden Ansprüche, wobei Furfural und 5-HMF und 2-Furansäure in einer kombinierten Menge von weniger als 5 Gew.-%, bezogen auf die Gesamttrockenmasse, im Nachbehandlungsschritt (POST) erzeugt werden, wie etwa als weniger als 3 Gew.-% bezogen auf die Gesamttrockenmasse im Nachbehandlungsschritt (POST), wie etwa weniger als 1 Gew.-% bezogen auf die Gesamttrockenmasse im Nachbehandlungsschritt (POST), wie etwa weniger als 0,5 Gew.-% bezogen auf die Gesamttrockenmasse im Nachbehandlungsschritt (POST), wie etwa weniger als 0,06 Gew.-% bezogen auf die Gesamttrockenmasse im Nachbehandlungsschritt (POST).

17. Verfahren nach einem der vorhergehenden Ansprüche, wobei das anaerobe Vergären (AD) der nachbehandelten festen Waschfraktion (PSW) für weniger als 30 Tage durchgeführt wird.

18. Verfahren nach einem der vorhergehenden Ansprüche, wobei Lignin (LIG) in dem nachbehandelten Biomassengärrest (PBD) oder getrockneten nachbehandelten Biomassengärrest (DPBD) in einer Menge von mehr als 30 Gew.-% enthalten ist, wie etwa in einer Menge von mehr als 35 Gew.-%, wie etwa in einer Menge von mehr als 40 Gew.-%.

19. Verfahren nach einem der vorhergehenden Ansprüche, wobei das Verfahren ferner Rückführung mindestens eines Teils einer flüssigen Fraktion aus einem beliebigen Trennschritt (SEP) zu einem beliebigen anaeroben Vergärungsschritt (AD) und/oder dem Vorbehandlungsschritt (PRE) umfasst.

## Revendications

1. Procédé de traitement de digestat de biomasse, le procédé comprenant les étapes de
- fourniture d'un digestat de biomasse (BD),
- soumission du digestat de biomasse à une étape de séparation (SEP) en une fraction liquide (LF) et une fraction de digestat de biomasse solide (SBD),
- soumission de la fraction de digestat de biomasse solide (SBD) à une étape de lavage acide (AW) à une pression inférieure à 200 kPa (2 bar) pour obtenir un digestat de biomasse lavé (WBD),
- séparation (SEP) du digestat de biomasse lavé (WBD) en une fraction de lavage liquide (LW) et une fraction de lavage solide (SW),
- post-traitement (POST) de la fraction de lavage solide (SW) pour obtenir une fraction de lavage solide post-traitée (PSW) et
- digestion anaérobie (AD) de la fraction de lavage solide post-traitée (PSW) pour obtenir un digestat de biomasse post-traité (PBD),
la quantité de phosphore dans la fraction de lavage solide (SW) étant réduite par rapport à la quantité de phosphore dans la fraction de digestat de biomasse solide (SBD),
ledit post-traitement étant choisi dans la liste constituée par une explosion à la vapeur, un traitement par laccase, un traitement par base, un traitement par acide dilué, un traitement thermique, une dissolution dans des liquides ioniques et une précipitation ultérieure, une dissolution dans des hydrates de sels fondus, ou un procédé organosolv, ou des combinaisons de ceux-ci.

2. Procédé selon la revendication 1, ladite étape de lavage acide (AW) étant non pressurisée.

3. Procédé selon la revendication 1 ou 2, ladite quantité de phosphore dans la fraction de lavage solide (SW) étant inférieure à 10 % en poids du phosphore dans le digestat de biomasse (BD).

4. Procédé selon l'une quelconque des revendications précédentes, le pH dans l'étape de lavage acide (AW) étant inférieur à 6,5, tel qu'inférieur à 6, tel qu'inférieur à 4.

5. Procédé selon l'une quelconque des revendications précédentes, la température dans l'étape de lavage acide (AW) étant comprise entre 50 et 100 degrés Celsius.

6. Procédé selon l'une quelconque des revendications précédentes, l'acide dans l'étape de lavage acide (AW) étant un acide fort présentant un pKa inférieur à 3 ou un acide faible présentant un pKa supérieur à 3.

7. Procédé selon l'une quelconque des revendications précédentes, ledit acide dans l'étape de lavage acide (AW) étant un acide organique.

8. Procédé selon l'une quelconque des revendications précédentes, ladite quantité de phosphore dans la fraction de lavage liquide (LW) étant réduite pour obtenir une fraction liquide à faible teneur en phosphore.

9. Procédé selon l'une quelconque des revendications précédentes, ladite quantité de phosphore dans la fraction de lavage liquide (LW) étant réduite en soumettant la fraction de lavage liquide (LW) à un échange d'ions (IEX).

10. Procédé selon l'une quelconque des revendications 1 à 8, ladite quantité de phosphore dans la fraction liquide de lavage (LW) étant réduite par précipitation avec une base.

11. Procédé selon l'une quelconque des revendications précédentes, ladite fraction de lavage liquide (LW) étant remise en circulation dans l'étape de lavage acide (AW).

12. Procédé selon l'une quelconque des revendications précédentes, ledit digestat de biomasse (BD) étant fourni par chauffage d'une biomasse molle (SB) dans une étape de prétraitement (PRE) suivie d'une digestion anaérobie (AD) de la biomasse molle (SB) pour obtenir le digestat de biomasse (BD).

13. Procédé selon l'une quelconque des revendications précédentes, ladite température de post-traitement (POST) étant comprise entre 40 et 230 degrés Celsius, telle qu'entre 40 et 60 degrés Celsius, telle qu'entre 80 et 160 degrés Celsius, telle qu'entre 150 et 230 degrés Celsius, telle qu'entre 50 et 120 degrés Celsius, telle qu'entre 90 et 140 degrés Celsius, telle qu'entre 130 et 220 degrés Celsius.

14. Procédé selon l'une quelconque des revendications précédentes, ladite étape de post-traitement (POST) étant réalisée à une pression comprise entre 1 MPa (10 bar) et 2 MPa (20 bar).

15. Procédé selon l'une quelconque des revendications précédentes, ladite température de post-traitement (POST) étant comprise entre 150 et 230 degrés Celsius, telle qu'entre 170 et 210 degrés Celsius, telle qu'entre 180 et 200 degrés Celsius.

16. Procédé selon l'une quelconque des revendications précédentes, du furfural et du 5-HMF et de l'acide 2-furoïque étant générés en une quantité combinée inférieure à 5 % en p/p par rapport à la matière sèche totale dans l'étape de post-traitement (POST), telle qu'inférieure à 3 % en p/p par rapport à la matière sèche totale dans l'étape de post-traitement (POST), telle qu'inférieure à 1 % en p/p par rapport à la matière sèche totale dans l'étape de post-traitement (POST), telle qu'inférieure à 0,5 % en p/p par rapport à la matière sèche totale dans l'étape de post-traitement (POST), telle qu'inférieure à 0,06 % en p/p par rapport à la matière sèche totale dans l'étape de post-traitement (POST).

17. Procédé selon l'une quelconque des revendications précédentes, ladite digestion anaérobie (AD) de la fraction de lavage solide post-traitée (PSW) étant effectuée pendant moins de 30 jours.

18. Procédé selon l'une quelconque des revendications précédentes, de la lignine (LIG) étant comprise dans le digestat de biomasse post-traité (PBD) ou le digestat de biomasse post-traité séché (DPBD) en une quantité supérieure à 30 % en poids, telle qu'en une quantité supérieure à 35 % en poids, telle qu'en une quantité supérieure à 40 % en poids.

19. Procédé selon l'une quelconque des revendications précédentes, ledit procédé comprenant en outre la remise en circulation d'au moins une partie d'une fraction liquide de toute étape de séparation (SEP) à toute étape de digestion anaérobie (AD) et/ou à l'étape de prétraitement (PRE).
